# EUROPEAN PATENT SPECIFICATION

(11) **EP 0 913 144 B1**
(45) Date of publication and mention of the grant of the patent: **17.11.2004**
(21) Application number: 98111869.8
(22) Date of filing: 25.06.1998
(51) Int. Cl.: A61K 7/16

(54) **Microtextured oral hygiene compositions**
Mikrotexturierte Mundpflegemittel
Compositions microtexturées pour l'hygiène buccale

(30) Priority: 26.06.1997 GB 9713403
(43) Date of publication of application: 06.05.1999
(73) Proprietor: The Boots Company PLC, Nottingham, Nottinghamshire NG2 3AA (GB)
(72) Inventor: Haywood, Julia Louise, Nottingham, NG2 3AA (GB); Nuthall, Trevor George, Nottingham, NG2 3AA (GB)
(74) Representative: Jones, Stephen Anthony

(56) References cited:
- EP-A- 0 543 442
- WO-A-95/34275
- FR-A- 1 600 227
- FR-A- 2 183 228
- US-A- 4 485 089

## Description

The present invention relates to oral hygiene compositions, particularly dentifrices. Dentifrices are used to clean the teeth and gums and to be acceptable to the consumer who uses them they must provide effective cleansing and exhibit an acceptable "mouthfeel". Mouthfeel is the sensation experienced by the user when the dentifrice is used. If the user finds that the mouthfeel is unpleasant, then he or she may be deterred from using the product as often as a good dental hygiene regime requires.

The present invention provides oral health compositions which provide effective cleansing and which exhibit a satisfactory mouthfeel.

Accordingly, the present invention provides oral hygiene compositions in which a combination of a) a friable agglomerated siliceous material and b) a particulate cellulose material having a particle size of less than 350 µm is used as a cleaning and polishing agent.

The friable agglomerated siliceous material is preferably prepared by agglomerating siliceous material of very small particle size to provide agglomerates with a particle size of less than 350 µm. The small particle size siliceous materials have been used in dentifrices as thickeners but are not known by those skilled in the art to have any cleaning properties when so used. The agglomerated siliceous materials used in the compositions of the present invention however provide, in combination with the particulate cellulose material, effective cleansing and acceptable mouthfeel. The agglomerated siliceous material may comprise from 1 to 10% preferably 2 to 4% most preferably about 3% of the oral health compositions of the present invention. The agglomerated siliceous materials may incorporate colouring agents. If such coloured agglomerated siliceous materials are incorporated into a clear gel oral hygiene composition then a visually attractive speckled effect is provided. Suitable agglomerated siliceous materials having a mean particle size of 310 µm are available commercially from Crosfield Limited under the trade designation Sorbisol BFG 50.

Suitably, the particulate cellulose material has a particle size of from about 1 µm to 350 µm, suitably from about 10 µm to about 100 µm, more suitably from about 20 µm to about 70 µm. A preferred cellulose cleaning/polishing agent comprises a high percentage of alpha-cellulose comprising 1,4-beta-glycosidically linked D-glucose molecules with a degree of polymerisation of about 500 or more.

Suitably, the particulate cellulose material comprises from about 0.5% to about 10% by weight, preferably from about 1% to about 6%, most preferably about 4% by weight of the oral hygiene compositions of the present invention.

It has been found that combinations of a friable agglomerated siliceous material with a particulate cellulose material having a particle size of less than 50 µm provide surprisingly effective cleaning action without causing damage to tooth enamel.

In a preferred embodiment of the present invention there is provided an oral hygiene composition comprising a combination of a friable agglomerated siliceous material, a particulate cellulose material having a particle size of less than 50 µm and an additional abrasive. In particular the cellulose particle size may be less than about 40 µm, for example about 35 µm.

Suitably, the additional abrasive is selected from: silica, alumina, insoluble metaphosphates, calcium carbonate, dicalcium phosphate (in dihydrate and anhydrous forms), calcium pyrophosphate, natural and synthetic clays, and particulate thermosetting polymerised resins selected from melamine-ureas, melamine-formaldehydes, urea-formaldehydes, melamine-urea-formaldehydes, cross-linked epoxides, melamines, phenolics, cross-linked polyesters and any compatible mixtures thereof.

Suitable, the composition comprises from about 0.1% to about 15% by weight, preferably from 1% to about 10%, suitably about 5% by weight of the additional abrasives. Suitably, about 98% or more of the additional abrasive has a particle size of less than about 45 µm, optionally between about 25 µm and about 45 µm.

Preferably, the non-cellulose abrasive is a particulate silica abrasive. Suitable silica abrasives include the hydrated silicas, particularly those available under the trade names 'Sident' from Degussa AG, 'Zeodent' from J M Huber Corporation, 'Syloblanc' from W R Grace or 'Sorbosil' from Crosfield Ltd.

It has been found that certain alkali metal salts enhance the cleaning and/or polishing action of the oral hygiene compositions of the present invention.

Therefore, in a further preferred embodiment of the present invention there is provided an oral hygiene composition comprising a friable agglomerated siliceous material, a particulate cellulose material having a particle size of less than 350 µm, and an agent to provide an enhanced cleaning/polishing action selected from one or more of: an alkali metal bicarbonate, an alkali metal orthophosphate, an alkali metal citrate and any compatible mixtures thereof. Such compositions are suitable for removing stains and/or plaque. The preferred amounts and particle sizes of the particulate cellulose in such compositions are as given for the compositions described above.

Preferably, the compositions of the present invention further comprise an alkali metal bicarbonate, suitably sodium bicarbonate. Such a bicarbonate may be included in an amount of from about 0.1% to about 10%, suitably from about 1% to about 5%, for example about 2% by weight. Inclusion of a bicarbonate provides an enhanced cleaning action.

Oral hygiene compositions according to the present invention may contain one or more additional ingredients such as, for example, humectants, surfactants, gelling agents, fluoride sources, desensitising agents, flavourings, colourings, sweeteners, preservatives, structuring agents, thickeners, bactericides, anti-tartar agents and antiplaque agents.

Suitable humectants include polyhydric alcohols such as xylitol, sorbitol, glycerol, propylene glycol and polyethylene glycols. Mixtures of glycerol and sorbitol are particularly effective. A humectant helps to prevent dentifrice compositions from hardening on exposure to air, and may also provide a moist feel, smooth texture, flowability, and a desirable sweetness in the mouth. Suitably, such humectants may comprise up to about 85% by weight, preferably up to about 60% by weight of the oral hygiene composition.

Suitable surfactants are usually water-soluble organic compounds, and may be anionic, non-ionic, cationic or amphoteric species. The surfactant used should preferably be reasonably stable and able to produce a foam in use.

Anionic surfactants include the water-soluble salts of C10-18 alkyl sulphates (eg sodium lauryl sulphates), water-soluble salts of C10-18 ethoxylated alkyl sulphates, water-soluble salts of C10-18 alkyl sarcosinates, the water-soluble salts of sulphonated monoglycerides of C10-18 fatty acids (eg sodium coconut monoglyceride sulphonates), alkyl aryl sulphonates (eg sodium dodecyl benzene sulphonate) and sodium salts of the coconut fatty acid amide of N-methyltaurine.

Non-ionic surfactants suitable for use in oral compositions include the products of the condensation of alkylene oxide groups with aliphatic or alkylaromatic species, and may be, for example, polyethylene oxide condensates of alkyl phenols, ethylene oxide/propylene oxide copolymers, available from BASF Wyandotte Chemical Corporation under the trade name 'Pluronic', ethylene oxide/ethylene diamine copolymers, ethylene oxide condensates of aliphatic alcohols, long chain tertiary amine oxides, long chain tertiary phosphine oxides, long chain dialkyl sulphoxides and mixtures thereof. Alternatives include ethoxylate sorbitan esters such as those available from ICI under the trade name 'Tween'.

Zwitterionic surfactants are generally derivatives of aliphatic quaternary ammonium, phosphonium, and sulphonium compounds, in which the aliphatic species may be branched or unbranched, and in which one of the aliphatic species is a C8-18 species and another contains an anionic hydrophilic group, such as carboxy, sulphonate, sulphate, phosphate or orthophosphonate.

Cationic surfactants are generally quaternary ammonium compounds having one C8-18 alkyl chain and include, for example, lauryl trimethylammonium chloride, cetyl trimethylammonium bromide, cetyl pyridinium chloride, cetyl di-isobutylphenoxyethoxyethyldimethylbenzylammonium chloride, coconutalkyltrimethylammonium nitrite and cetyl pyridinium fluoride. Also useful are benzyl ammonium chloride, benzyl dimethyl stearylammonium chloride, and tertiary amines having one C1-18 hydrocarbon group and two (poly)oxyethylene groups.

Amphoteric surfactants are generally aliphatic secondary and tertiary amines comprising aliphatic species which may be branched or unbranched, and in which one of the aliphatic species is a C8-18 species and the other contains an anionic hydrophillic group, for example, sulphonate, carboxylate, sulphate, phosphonate or phosphate. Examples of quaternary ammonium compounds are the quaternized imidazole derivatives available under the trade name 'Miranol' from the Miranol Chemical Company.

Suitably, the surfactant is included in an amount of up to about 20% by weight, preferably up to about 10% by weight of the oral hygiene composition.

Structuring agents may be required in, for example, dentifrices and gums to provide desirable textural properties and "mouthfeel". Suitable agents include natural gum binders such as gum tragacanth, xanthan gum, gum karaya and gum arabic, seaweed derivatives such as Irish moss and alginates, smectite clays such as bentonite or hectorite, carboxyvinyl polymers and water-soluble cellulose derivatives such as hydroxyethyl cellulose and sodium carboxymethyl cellulose. Improved texture may also be achieved, for example, by including colloidal magnesium aluminium silicate. Suitably, the structuring agent is included in an amount of up to about 5% by weight, preferably up to about 3% by weight of the oral hygiene composition.

Suitable thickeners include the silicas which are well known in the art for this purpose, for example those silicas sold under the trade name Sorbisol by Crosfield Ltd.

Fluoride sources suitable for use in oral hygiene compositions of the present invention include sodium fluoride, zinc fluoride, potassium fluoride, aluminium fluoride, lithium fluoride, sodium monofluorophosphate, acidulated phosphate fluoride, stannous fluoride, ammonium fluoride, ammonium bifluoride and amine fluoride.

Preferably the fluoride source is present in an amount sufficient to provide from about 50 ppm to about 4,000 ppm fluoride ions in the composition. Inclusion of a fluoride source is beneficial, since fluoride ions are known to become incorporated into the hydroxyapatite of tooth enamel, thereby increasing the resistance of the enamel to decay. Fluoride is also now thought to act locally on the tooth enamel, altering the remineralisation - demineralisation balance in favour of remineralisation. Inclusion of a fluoride source is also desirable when a polyphosphate anticalculus agent is included, in order to inhibit the enzymic hydrolysis of such polyphosphates by salivery phosphatase enzymes.

Suitable desensitising agents include, for example, formaldehyde, potassium nitrate, potassium chloride and strontium chloride (suitably as hexahydrate), strontium acetate (suitably as hemihydrate) and sodium citrate/Pluronic gel.

Flavouring agents may be added to increase palatability and may include, for example, levo-methol, oils of peppermint, spearmint, wintergreen, sassafras and clove. Sweetening agents may also be used, and these include D-tryptophan, saccharin dextrose, aspartame, levulose, acesulfam, dihydrochalcones and sodium cyclamate.

Typically, such flavouring agents are included in amounts of up to about 5% by weight, preferably up to about 2% by weight of the oral hygiene composition. Colouring agents and pigments may be added to improve the visual appeal of the composition. Suitable colourants include dyes, such as FD & C blue No. 1, D & C yellow No. 10 and D & C yellow No. 3. A suitable and commonly used pigment is titanium dioxide, which provides a strong white colour.

Suitably, the compositions of the invention may include an antimicrobial agent as a preservative and/or anti-plaque agent. Suitable antimicrobial agents include zinc salts such as zinc citrate, cetyl pyridinium chloride, aliphatic amines, bis-biguanides, such as chlorhexidine, bromochlorophene, hexachlorophene, salicylanilides, quaternary ammonium compounds and triclosan. Enzymic systems providing a source of a natural biocide may be used as alternatives to or in combination with the biocides listed. For example, a system comprising lactoperoxidase and glucose oxidase may be used to generate hydrogen peroxide in the presence of glucose, water and oxygen.

The composition may also comprise an anti-calculus agent. Suitable anti-calculus agents include polyphosphates, preferably pyrophosphates. A preferred source of pyrophosphate is a mixture of tetrasodium pyrophosphate and tetrapotassium pyrophosphate. Suitably, the ratio of tetrasodium pyrophosphate to tetrapotassium pyrophosphate is 0:1 to 3:1, preferably 0:1 to 1:1. Preferably, tetrapotassium pyrophosphate is the predominant species.

The present invention will be illustrated with reference to the following non-limiting Examples.

### Example 1

A toothpaste is prepared to the following composition:

| Ingredient | % w/w |
|---|---|
| Sorbitol (70%) | 40.00 |
| Sodium fluoride BP | 0.24 |
| Silica (abrasive) [Sorbosil AC 35] | 8.33 |
| Silica (thickener) [Sorbosil TC 15] | 4.56 |
| Microgranules (green) [Sorbisol BFG 50] | 2.5 |
| Microgranules (white) [Sorbisol BFG 50] | 0.5 |
| Cellulose abrasive | 4 |
| Sodium saccharin | 0.26 |
| Sodium bicarbonate | 2 |
| A mixture of sodium lauryl sulphate and sodium sulphate (available under the trade name 'Empicol LZ PDR) | 1.39 |
| Titanium dioxide | 0.50 |
| Sodium carboxymethylcellulose | 0.90 |
| Zinc citrate trihydrate | 0.50 |
| Polyethylene glycol 1500 (PEG 32) | 1.39 |
| Bromochlorophene | 0.1 |
| Flavour | 0.92 |
| Purified water | to 100 |

### Method of manufacture

1. To purified water add polyethylene glycol and dissolve sodium fluoride and sodium saccharin.
2. Add sorbitol and mix thoroughly.
3. Add:
   silica (abrasive), silica (thickener), microgranules, cellulose abrasive, sodium carboxymethylcellulose, sodium bicarbonate, titanium dioxide, zinc citrate trihydrate, the mixture of sodium lauryl sulphate and sodium sulphate.
   Mix thoroughly under vacuum until smooth paste is formed.
4. Dissolve bromochlorophene in flavour oils and add this solution to the paste under vacuum.
5. Mix under vacuum until a smooth homogeneous paste is produced.

### Example 2

In a similar manner to that described in Example 1 the following toothpaste formulation is made

| | |
|---|---|
| Sorbitol (70%) | 45 |
| Sodium Saccharin | 0.26 |
| Sodium Monofluorophosphate | 0.84 |
| Sodium Carboxymethylcellulose | 0.85 |
| Silica (abrasive)[Zeodent 113] | 6 |
| Silica (thickener)[Zeodent 163] | 8.33 |
| Cellulose Abrasive | 4 |
| Microgranules (blue)[Sorbosil BFG51] | 0.5 |
| Microgranules (white)[Sorbosil BFG50] | 2.5 |
| Zinc Citrate | 0.5 |
| Titanium Dioxide | 0.75 |
| Sodium Bicarbonate | 2 |
| Bromochlorophene | 0.1 |
| A mixture of sodium lauryl sulphate and sodium sulphate (Empicol LZ Pdr) | 1.5 |
| Flavour | 0.92 |
| Purified water | to 100 |

### Example 3

In a similar manner to that described in Example 1 the following toothpaste formulation is made

| | |
|---|---|
| Sorbitol (70%) | 50 |
| Sodium saccharin | 0.26 |
| Sodium monofluorophophate | 0.84 |
| Sodium carboxymethylcellulose | 0.9 |
| Silica(abrasive)[Sident 9] | 6.94 |
| Silica(thickener)[Sident 22S] | 7.64 |
| Microganules (blue)[Sorbosil BFG51] | 0.5 |
| Microgranules (white)[Sorbosil BFG50] | 2.5 |
| Cellulose abrasive | 4 |
| Zinc citrate | 0.5 |
| Titanium dioxide | 0.75 |
| Sodium bicarbonate | 2 |
| Bromochlorophene | 0.1 |
| Flavour | 0.92 |
| A mixture of sodium lauryl sulphate and sodium sulphate (Empicol LZ Pdr) | 1.5 |
| Purified water | to 100 |

### Example 4

In a similar manner to that described in Example 1 the following toothpaste formulation is made

| | |
|---|---|
| Sorbitol (70%) | 45 |
| Silica thickener (Sident 22S) | 6.94 |
| Silica abrasive (Sident 9) | 5 |
| Cellulose abrasive | 5 |
| Microgranules (blue)[Sorbosil BFG51] | 0.5 |
| Microgranules (white)[SorbosilBFG50] | 2.5 |
| Sodium carboxymethylcellulose | 0.7 |
| Sodium saccharin | 0.26 |
| Titanium dioxide | 0.75 |
| Sodium fluoride | 0.24 |
| Zinc citrate | 0.5 |
| Bromochlorophene | 0.1 |
| Sodium bicarbonate | 2 |
| Flavour | 0.92 |
| Purified water | to 100 |

## Claims

1. Oral hygiene composition comprising a combination of
a) a siliceous material and
b) a particulate cellulose material having a particle size of less than 350 µm as a cleaning and polishing agent,
**characterized in that** the siliceous material is in friable agglomerated form.

2. Oral hygiene composition as claimed in claim 1 in which the agglomerated siliceous material comprises 1 to 10% of the composition and the particulate cellulose material comprises 0.5 to 10% of the composition.

3. Oral hygiene composition as claimed in claim 1 in which the agglomerated siliceous material comprises 2 to 4% of the composition and the particulate cellulose material comprises 1 to 6% of the composition.

4. Oral hygiene composition as claimed in any preceding claim in which the particulate cellulose material has a particle size of from about 1 µm to 350 µm.

5. Oral hygiene composition as claimed in claim 4 wherein the particulate cellulose material has a particle size of less than 50 µm.

6. Oral hygiene composition as claimed in claim 5 which comprises an additional abrasive selected from silica, alumina, insoluble metaphosphates, calcium carbonate, dicalcium phosphate (in dihydrate and anhydrous forms), calcium pyrophosphate, natural and synthetic clays, and particulate thermosetting polymerised resins selected from melamine-ureas, melamine-formaldehydes, urea-formaldehydes, melamine-urea- formaldehydes, cross-linked epoxides, melamines, phenolics, cross-linked polyesters and any compatible mixtures thereof.

7. Oral hygiene composition as claimed in any preceding claim which comprises an additional cleaning/polishing agent selected from one or more of: an alkali metal bicarbonate, an alkali metal orthophosphate, an alkali metal citrate and any compatible mixtures thereof.

8. Oral hygiene composition as claimed in any preceding claim comprising one or more additional ingredients selected from humectants, surfactants, gelling agents, fluoride sources, desensitising agents, flavourings, colourings, sweeteners, preservatives, structuring agents, thickeners, bactericides, anti-tartar agents and antiplaque agents.

## Patentansprüche

1. Mundpflegemittel, das eine Verbindung aus
a) einem silikathaltigen Stoff und
b) einem speziellen Zellulose-Stoff, der als Reinigungs- und Polierwirkstoff eine Partikelgröße von weniger als 350 µm besitz,
umfasst, **dadurch gekennzeichnet, dass** der silikathaltige Stoff in der Form eines unbeständigen Agglomerats ist.

2. Mundpflegemittel nach Anspruch 1, wobei der agglomerat-förmige, silikathaltige Stoff 1 bis 10% der Zusammensetzung umfasst und der spezielle Zellulose-Stoff 0,5 bis 10% der Zusammensetzung umfasst.

3. Mundpflegemittel nach Anspruch 1, wobei der agglomerat-förmige, silikathaltige Stoff 2 bis 4% der Zusammensetzung umfasst und der spezielle Zellulose-Stoff 1 bis 6% der Zusammensetzung umfasst.

4. Mundpflegemittel nach einem der vorhergehenden Ansprüche, wobei der spezielle Zellulose-Stoff eine Partikelgröße von ungefähr 1 µm bis 350 µm besitzt.

5. Mundpflegemittel nach Anspruch 4, wobei der spezielle Zellulose-Stoff eine Partikelgröße von weniger als 50 µm besitzt.

6. Mundpflegemittel nach Anspruch 5, das ein zusätzliches Poliermittel, ausgewählt von Silikat, Aluminiumoxid, unlöslichen Metaphosphaten, Kalziumkarbonat, Dikalzium-Phosphaten (in Dihydrat-Formen und wasserfreien Formen) Kalziumpyrophosphaten, natürlichen und synthetischen Tonmineralien, und speziellen thermostatoplastischen polymerisierten Harzen, ausgewählt von Melamin-Karbaniden, Melamin-Formaldehyden, Karbanid-Formaldehyden, Malamin-Karbanid-Formaldehyden, vernetzen Epoxide, Melaminen, Phenolen, vernetzen Polyester und deren möglichen Mischungen, umfasst.

7. Mundpflegemittel nach einem der vorhergehenden Ansprüche, das einen zusätzlichen Reinigungs- und Polierwirkstoff, der von einem oder mehreren: ein alkalimetallisches Bikarbonat, ein alkalimetallisches Orthophosphat, ein alkalimetallisches Zitrat und deren möglichen Mischungen, ausgewählt ist, umfasst.

8. Mundpflegemittel nach einem der vorhergehenden Ansprüche, wobei es einen zusätzlichen Bestandteil oder mehrere zusätzliche Bestandteile, die von Feuchthaltemitteln, Tensiden, Gelierwirkstoffen, Fluorid-Ausgangsstoffen, unempfindlich machenden Wirkstoffen, Geschmacksstoffen, Farbstoffen, Süßstoffen, Konservierungsstoffen, Struktur-Wirkstoffen, Verdickungsmitteln, Bakteriziden, Anti-Zahnstein-Wirkstoffen und Anti-Zahnbelag-Wirkstoffen ausgewählt sind, umfasst.

## Revendications

1. Composition pour l'hygiène buccale comprenant une combinaison d'
a) un matériau siliceux et
b) un matériau cellulosique sous forme de particules, les particules ayant une taille inférieure à 350 µm, en tant qu'agent de nettoyage et de polissage,
caractérisée en que le matériau siliceux se trouve sous forme d'un aggloméré friable.

2. Composition pour l'hygiène buccale selon la revendication 1 dans laquelle le matériau siliceux aggloméré représente de 1 à 10% de la composition et le matériau cellulosique sous forme de particules représente de 0,5 à 10% de la composition.

3. Composition pour l'hygiène buccale selon la revendication 1 dans laquelle le matériau siliceux aggloméré représente de 2 à 4% de la composition et le matériau cellulosique sous forme de particules représente de 1 à 6% de la composition.

4. Composition pour l'hygiène buccale selon l'une quelconque des revendications précédentes dans laquelle les particules du matériau cellulosique sous forme de particules ont une taille entre à peu près 1µm et 350 µm.

5. Composition pour l'hygiène buccale selon la revendication 4 dans laquelle les particules du matériau cellulosique sous forme de particules ont une taille inférieure à 50 µm.

6. Composition pour l'hygiène buccale selon la revendication 5 qui comprend un abrasif additionnel choisi parmi la silice, l'alumine, les métaphosphates insolubles, le carbonate de calcium, le phosphate de dicalcium (sous forme de dihydrate et d'anhydre), le pyrophosphate de calcium, les argiles naturelles et synthétiques et les résines polymérisées thermodurcissables sous forme de particules choisies parmi les mélamine-urées, les mélamine-formaldéhydes, les urée-formaldéhydes, les mélamine-urée-formaldéhydes, les époxydes réticulés, les mélamines, les phénoliques, les polyesters réticulés et un mélange compatible quelconque de ceux-ci.

7. Composition pour l'hygiène buccale selon l'une quelconque des revendications précédentes qui comprend un agent de nettoyage/polissage additionnel choisi parmi l'un ou plusieurs des suivants: un bicarbonate de métal alcalin, un orthophosphate de métal alcalin, un citrate de métal alcalin et un mélange compatible quelconque de ceux-ci.

8. Composition pour l'hygiène buccale selon l'une quelconque des revendications précédentes qui comprend un ou plusieurs ingrédients additionnels choisis parmi les humectants, les agents tensioactifs, les agents de gélification, les sources de fluorure, les agents désensibilisateurs, les substances aromatiques, les colorants, les édulcorants, les conservateurs, les agents structurants, les épaississants, les bactéricides, les agents anti-tartre et les agents anti-plaque.
